# EUROPEAN PATENT SPECIFICATION

(11) **EP 1 732 624 B1**
(45) Date of publication and mention of the grant of the patent: **15.10.2008**
(21) Application number: 05713052.8
(22) Date of filing: 09.02.2005
(51) Int. Cl.: A61M 5/142, A61M 5/145

(54) **WATERPROOF PORTABLE INFUSION DEVICE HAVING A CASING WITH MULTIPLE CROSS-VENTED SEALED HOUSINGS**
WASSERFESTE TRAGBARE INFUSIONSVORRICHTUNG MIT EINEM GEHÄUSE MIT MEHREREN ÜBER KREUZ BELÜFTETEN VERSCHLOSSENEN GEHÄUSEN
DISPOSITIF DE PERFUSION PORTATIF ETANCHE A L'EAU ET POURVU D'UNE ENVELOPPE COMPORTANT DE MULTIPLES CORPS SCELLES AERES TRANSVERSALEMENT

(30) Priority: 10.02.2004 US 774487
(43) Date of publication of application: 20.12.2006
(73) Proprietor: Animas Corporation, West Chester PA 19380 (US)
(72) Inventor: PAUL, Patrick, J., Downingtown, PA 19335 (US); O'CONNOR, Sean, Michael, West Chester, PA 19380 (US)
(74) Representative: Tunstall, Christopher Stephen
(86) International application number: PCT/US2005/003870
(87) International publication number: WO 2005/077438

(56) References cited:
- US-A- 5 718 562
- US-A1- 2002 169 439
- US-A1- 2003 163 090
- US-A1- 2003 187 525
- US-B1- 6 248 093

## Description

### BACKGROUND OF THE INVENTION

### Field of the Invention

The present invention relates to an external infusion device, such as a portable insulin pump, and more specifically to an external infusion device, such as a portable insulin pump, having a casing with multiple hermetically-sealed housings.

### Description of the Related Art

Portable external infusion devices, such as portable insulin pumps, are well-known. Users, such as diabetics, wear these devices in their clothing, *e.g.*, on a belt or in a clothing pocket. In order to allow the user to enjoy a full range of activities, including for example, swimming, and outdoor activities, it is necessary for the device to resist ingress of water, which could damage the device's internal electronic components.

The need for such water hermeticity is complicated by an additional need to ensure pressure equilibrium between the interior of the device and atmosphere, in order to avoid pressure gradients inside the device that could adversely impact the delivery of liquid medication, such as insulin. A need for rapid pressure equalization can arise, for example, when the user flies in an airplane, and pressure in the airplane cabin fluctuates due to ascent or descent of the airplane. Such a fluctuation in cabin pressure could cause pressure inside an insulin pump casing to rapidly exceed cabin pressure, which could result in a sudden unexpected and undesirable infusion of insulin to the user.

Conventional infusion pumps typically include a casing defining a single housing. The housing encloses, within a single external wall, a medicinal reservoir, a driving mechanism, electronic circuitry for controlling the driving mechanism, a battery, o-rings sealing a battery door and a reservoir door, and vents, to allow passage of air, but prevent passage of liquid. These vents allow pressure within the casing to equalize with atmospheric pressure.

Notwithstanding these features, the conventional single housing device has at least one major drawback, namely that ingress of water, spillage of insulin, or any other ingress of liquid, due to a mechanical failure, or an operator error, *e.g*., forgetting to securely shut the reservoir door or battery door after changing the reservoir or the battery, allows liquid to reach electric components and the sensitive electronic circuitry, which can damage the components and circuitry permanently, or at least cause the device to malfunction.

Moreover, while some known infusion pumps include a casing with separate compartments, these compartments are not hermetically sealed from one another, so water leaking into one compartment also can flow into the other compartment(s), with the same risk to electronic components and circuitry.

### SUMMARY OF THE INVENTION

Accordingly, the present invention is directed to an infusion device and a casing for an external infusion device that mitigates or substantially obviates one or more problems associated with limitations and disadvantages of the related art. Additional features and advantages of the invention will be set forth in the description which follows, and in part will be apparent from the description, or may be learned by practice of the invention. The advantages of the invention will be realized and attained by the apparatus particularly pointed out in the written description, drawings, and claims below.

To achieve these and other advantages, and in accordance with the purpose of the invention, as embodied and broadly described herein, the invention comprises an infusion device, *e.g*., a portable insulin pump, as recited in the claims.

Preferably, the first, or reservoir housing, is accessible by the user via a first opening in the casing outer wall, for refilling or replacing the reservoir. The third, or battery housing is preferably accessible by the user via a second opening in the casing outer wall for recharging or replacing the battery or batteries. Preferably, the second or electronics and mechanical housing is not accessible by the user.

Each of the primary vents includes a hydrophobic barrier, such as a hydrophobic membrane, which permits passage of air or gas, and permits pressure equalization, but prevents passage of liquid.

The secondary vents also include a hydrophobic barrier as described above.

Preferably, the hydrophobic membranes are selected so that a water entry pressure exceeds a pressure to which the membrane will be subjected upon immersion in liquid, and so that an airflow rate is as high as possible, to permit rapid pressure equalization.

It is to be understood that both the foregoing general description and the following detailed description are exemplary and explanatory and are intended to provide further explanation of the invention as claimed.

The accompanying drawings are included to provide a further understanding of the invention. They are incorporated in and constitute a part of the specification, illustrate a presently preferred embodiment of the invention, and together with the specification, serve to explain the principles of the invention.

### BRIEF DESCRIPTION OF THE DRAWINGS

Fig. 1 is a schematic depiction of a multiple-housing casing for an external infusion device in accordance with the present invention;

Fig. 2 is a schematic depiction of a multiple-housing casing in accordance with the invention, depicting ventilation of the housings via primary and secondary vents;

Fig. 3 is a schematic depiction of a multiple-housing casing in accordance with an invention, depicting ventilation in a first abnormal situation comprising occlusion of the reservoir housing primary vent;

Fig. 4 is a schematic depiction of a multiple-housing casing in accordance with the invention, depicting ventilation in a second abnormal situation, comprising occlusion of the battery housing primary vent;

Fig. 5 is a schematic depiction of a multiple-housing casing in accordance with the invention, depicting ventilation in a third abnormal situation, comprising occlusion of a reservoir housing secondary vent;

Fig 6 is a schematic depiction of a multiple-housing casing in accordance with the invention, depicting ventilation in a fourth abnormal situation, comprising occlusion of a battery housing secondary vent;

Fig. 7 is a top perspective view of an external infusion device casing in accordance with the invention;

Fig. 8 is a front view of the external infusion device casing of Fig. 7;

Fig. 9 is a schematic depiction of a conventional single housing for an external infusion pump.

### DETAILED DESCRIPTION OF THE PREFERRED EMBODIMENTS

Reference will now be made in detail to the present preferred embodiment of the invention, an example of which is illustrated in the accompanying drawings.

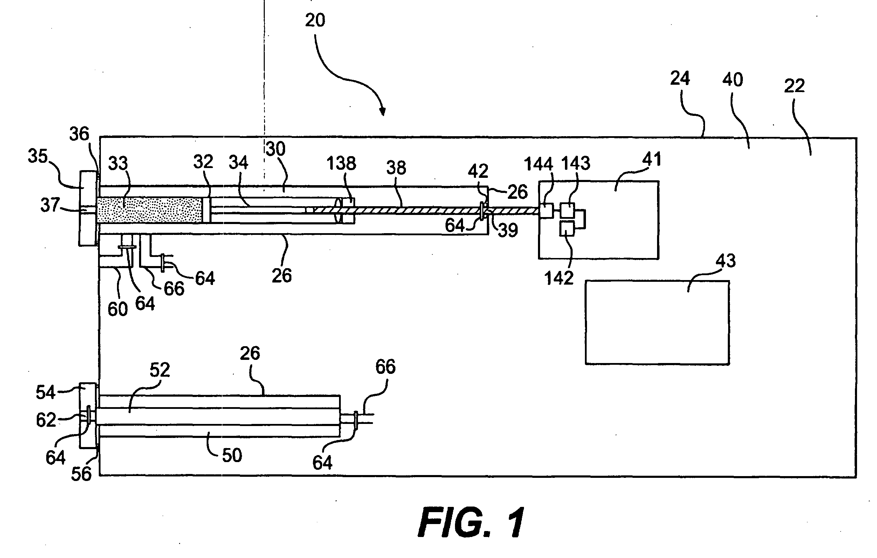

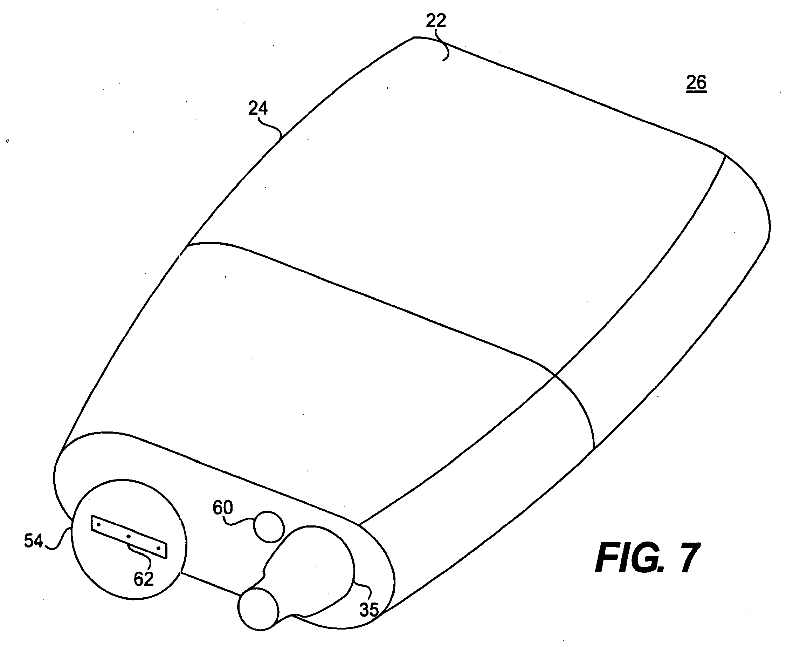

As embodied herein and referring to Figs. 1 and 7, an external infusion device 20 includes a multiple-housing casing 22.

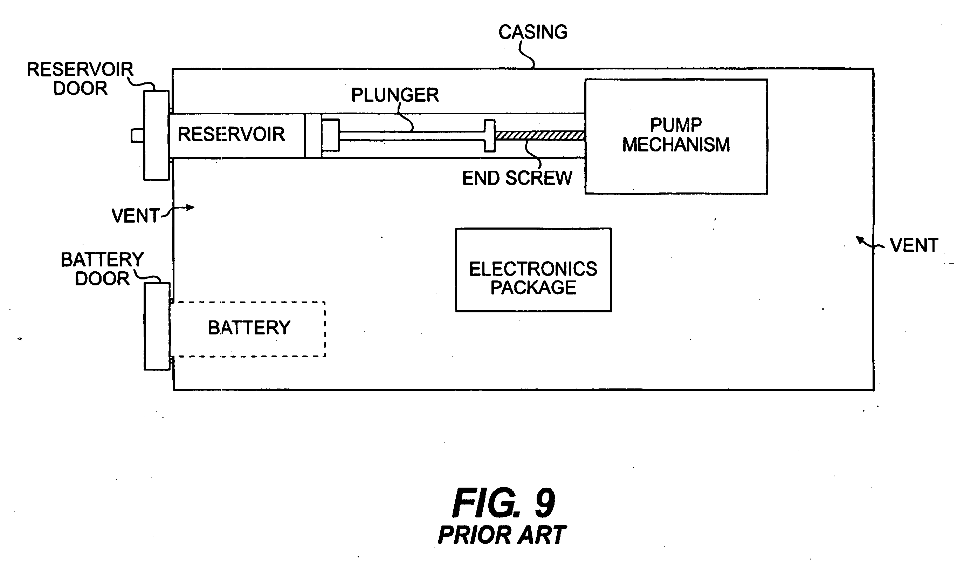

In accordance with the invention, an external wall 24 surrounds a plurality of housings, discussed below, for enclosing various components of the external infusion device, respectively the housings being separated by a series of internal walls 26 into separate housings that are hermetically sealed and substantially isolated from one another, the hermetically-sealed housings being joined together. As embodied herein, and referring to Figs. 1 and 9, a first or "reservoir" housing 30 is defined by internal walls 26 within the external wall 24. Reservoir housing 30 is configured to hold a refillable, or alternatively replaceable, liquid reservoir 32. Reservoir 32 includes a generally cylindrical storage portion 33, terminating in a reduced-diameter tip 37, the storage portion 33 being configured to hold medicinal liquid, *e.g*., insulin. Reservoir 32 also includes a linear-moving plunger 34. Storage portion 33 and plunger 34 cooperate to define a syringe structure, as is well-known in the art. Preferably, reservoir housing 30 includes an opening, closed by a screw-on cap 35, mounted in external wall 24, to give the user of the device access to reservoir compartment 30 in order to refill or replace the reservoir 32 as necessary. It is further preferred that a sealing o-ring 36 be provided to form a seal between the reservoir 32 and the walls of reservoir housing 30. It is also preferred that screw-on cap 35 can be configured with a port 37, to allow flow of the liquid, *e.g*., the insulin, out of the reservoir 32 and, via a cannula (not shown), to the user.

As embodied herein, reservoir housing 30 also includes a driving mechanism, preferably a lead screw 38 and a drive nut 138, as broadly shown in Fig. 1, for applying pressure to the plunger 34, and thereby to the liquid in the storage portion 33 of reservoir 32, to dispense the liquid therein out through the port 37. Lead screw 38 rotates, and its rotational motion acts on the drive nut 138, which translates the rotational motion to linear motion, and pushes in a linear direction against plunger 34. Lead screw 38 passes through an aperture 39 in the internal wall 26 to a pump mechanism, which will be described below.

In accordance with the invention, and as embodied herein, referring to Fig. 1, a second or electronics and mechanical housing 40 is provided within external wall 24. Electronics and mechanical housing 40 encloses a pump mechanism 41, which as broadly embodied herein, includes a motor 142, preferably a brushless dc motor, a planetary gear box 143, and a speed-reducing gear train 144, for transmitting power from the motor 142 to the base of the lead screw 38. A seal 42 is provided in aperture 39, which with the lead screw 38, seals the aperture 39 from water leakage, while still allowing passage through the aperture 39 of the lead screw 38. Seal 42 preferably is a rotational ball seal, which opposes passage of water through the aperture 39, while reducing friction acting on lead screw 38. Electronics and mechanical housing 40 further encloses an electronics package 43, including, *e.g*., a controller, a memory, a transmitter/receiver for transmitting and receiving wireless data and control signals to and from an external control source, and associated electronics for interacting with a keypad and a display (not shown) on external wall 24. The pump mechanism 41, the lead screw 38 and the plunger 34 function together to dispense the liquid from the reservoir 32 to the user according to a selected pattern. For example, in the case where the external infusion device 20 is an insulin pump, and the reservoir 32 contains liquid insulin, electronics package 43 may be programmed with one or more basal patterns governing delivery of insulin to the user in accordance with, e.g., the user's measured blood glucose level, the user's ingestion of food, the user's level of exercise, and so on, as is well known in the field of diabetes treatment. These basal patterns can be programmed into the controller in the electronics package 43 either locally, or from the external control source. As embodied herein, the electronics and mechanical housing 40 is not accessible by the user.

In accordance with the invention, and as embodied herein, referring to Fig. 1, a third or "battery" housing 50 is provided inside external wall 24. Battery housing 50 is configured to enclose a battery 52 or else multiple batteries 52. The precise type of battery is not essential to the invention. Preferably battery housing 50 includes an opening, sealed by a door 54, removably or pivotally mounted in external wall 24, to give the user access to the battery housing 50 in order to replace or recharge the battery or batteries 52 as needed, and it is further preferable that door 54 include a sealing o-ring 56.

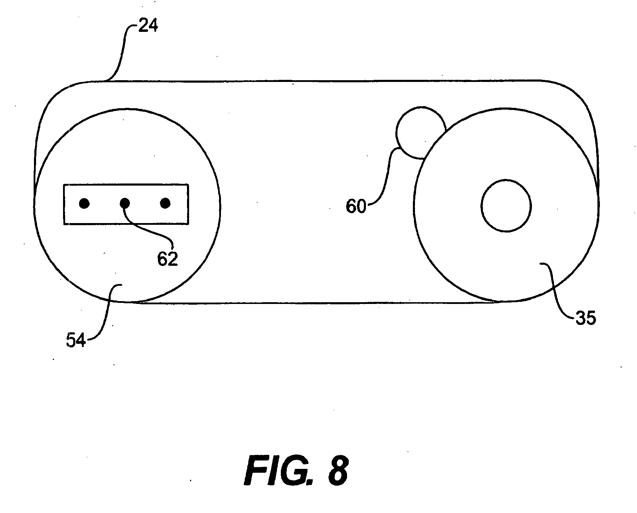

In accordance with the invention, a plurality of primary vents are provided for venting the infusion device to the atmosphere. As embodied herein, and referring to Figs. 1, 7, and 8, a first primary vent 60 is provided in external wall 24, with an opening into the reservoir housing 30. First primary vent 60 vents to atmosphere the reservoir housing 30 in order to ensure there is no differential pressure between the plunger 34 and atmosphere, which could result in an inadvertent driving force being applied to the liquid in reservoir 32 capable of dispensing the liquid. As embodied herein, a second primary vent 62 is provided, preferably in battery housing door 54, opening into battery housing 50. Second primary vent 62 vents to atmosphere the battery housing 50 in order to prevent uncontrolled pressure build-up in the compartment resulting from gas buildup, *e.g*., hydrogen gas resulting from a chemical reaction in the battery.

Preferably both of the first and second primary vents 60 and 62 include an aperture, sealed with a hydrophobic barrier, such as a hydrophobic membrane 64, such membranes being well known in the art. Hydrophobic membranes allow air and other gases to pass therethrough, thereby allowing pressure to equalize across each primary vent, but will prevent passage of water therethrough.

As embodied herein, no primary vent is provided directly between the electronics and mechanical housing 40 and the atmosphere; nevertheless, venting of electronics and pump housing 40 is accomplished in the manner discussed below.

In accordance with the invention, at least one secondary vent is provided between selected ones of the compartments. As embodied herein, each secondary vent also includes a hydrophobic barrier, e.g., a hydrophobic membrane.

As embodied herein, and as shown in Figs. 1-6, a secondary vent 66, including a hydrophobic membrane 64, similar to the hydrophobic membranes used in primary vents 60 and 62 is provided in internal wall 26, between the electronics and mechanical housing 40 and the reservoir housing 30, between the electronics and mechanical housing 40 and the battery housing 50, or both. Secondary vent or vents 66 prevent an undesired buildup of pressure in the electronics and mechanical housing 40, thereby preventing condensation within the compartment, and ensuring an adequate back pressure on the keypad (not shown) on external wall 24. Secondary vent or vents 66 allow the electronics and mechanical housing 40 to vent to atmosphere via the primary vent 60 or 62, in the respective reservoir housing 30 or battery housing 50, or both.

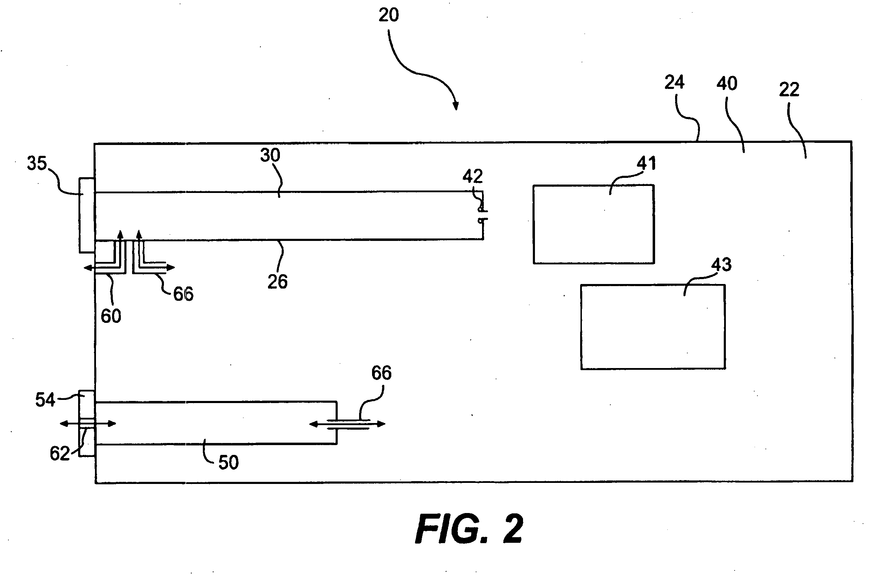

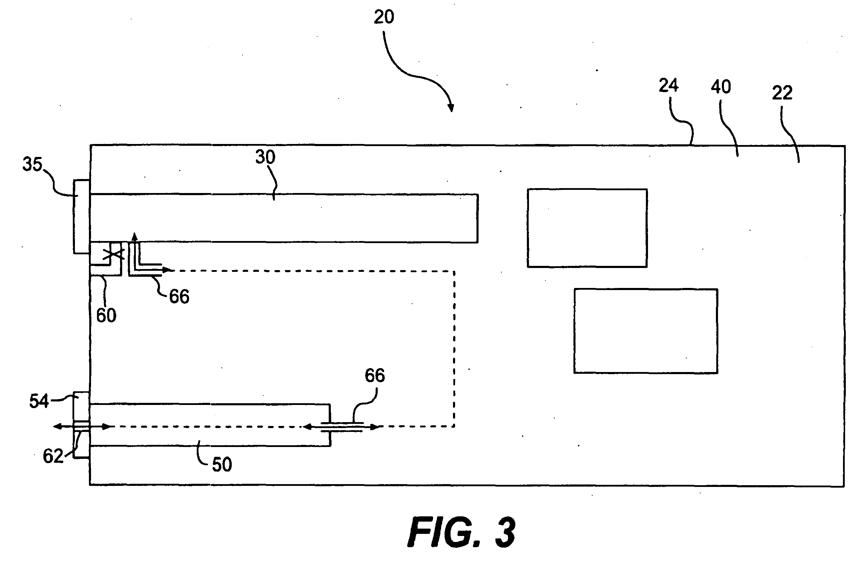

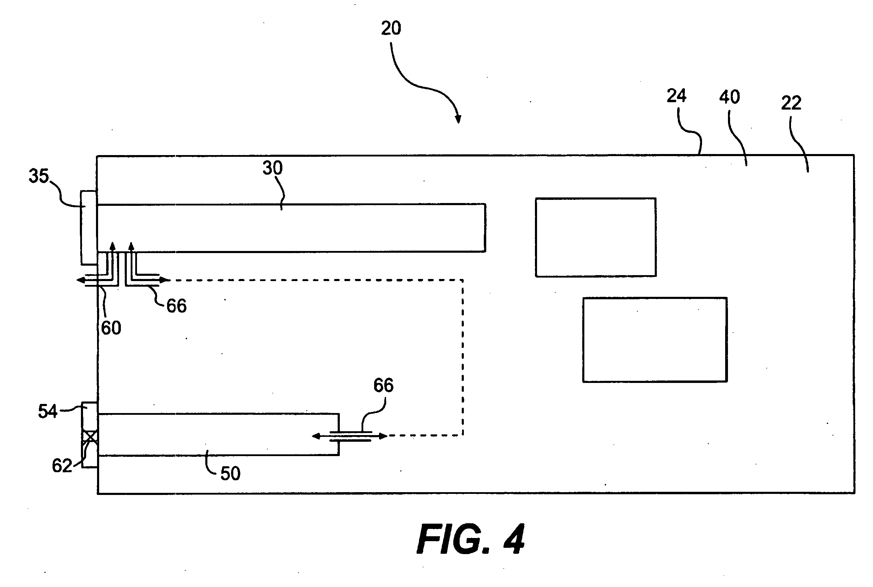

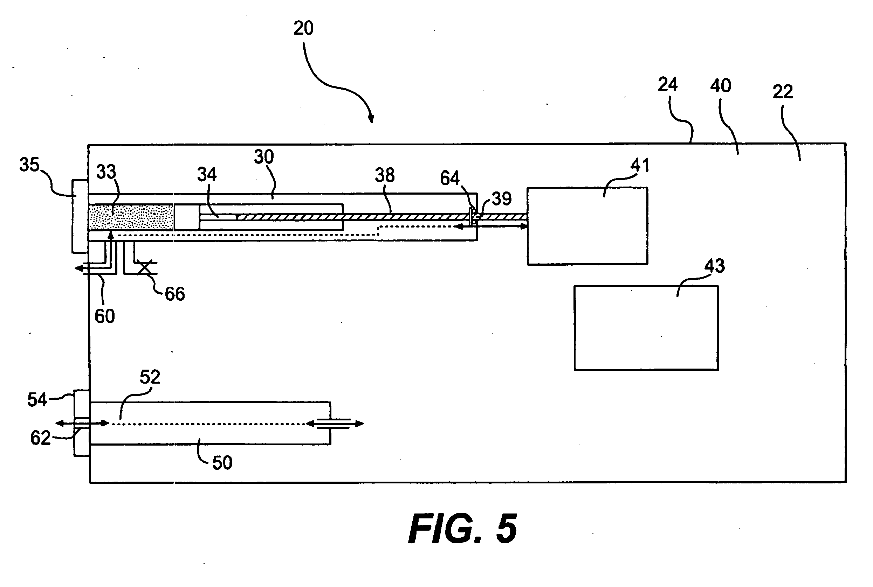

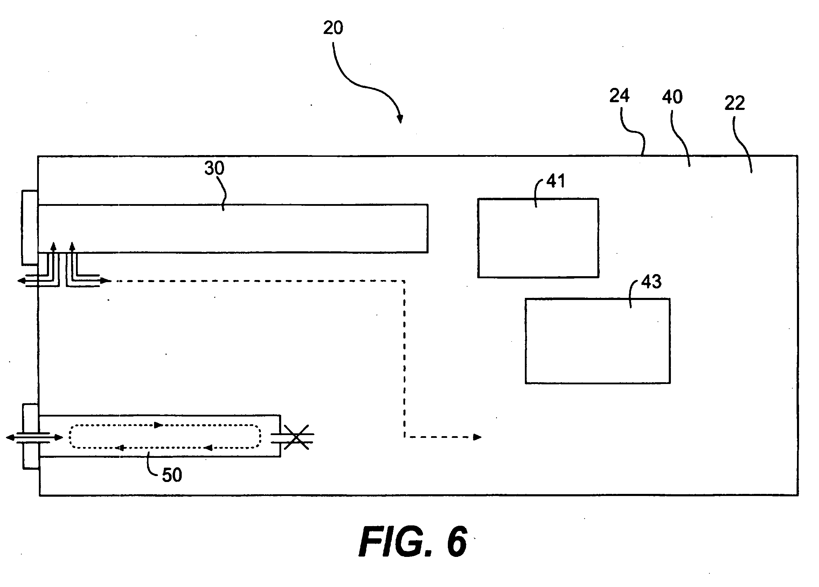

As embodied herein, redundancy created by the presence of primary vents 60 and 62 and secondary vent or vents 66, shown in Fig. 2, ensures venting and pressure equalization of all three housings, even during abnormal situations, such as, for example, occlusion of the reservoir housing primary vent 60 (shown, *e.g*., in Fig. 3), occlusion of the battery housing primary vent 62 (shown, *e.g*., in Fig. 4), or occlusion of one of the secondary vents 66 (shown, *e.g*., in Figs. 5 and 6).

Moreover, as embodied herein, if the battery housing 50, and/or the reservoir housing 30 were to inadvertently fill with liquid because of (a) the mechanical failure of one or both o-rings 36 or 56; (b) the user's failure to secure one or both housing doors 35 or 54; or (c) failure of the hydrophobic membrane 64 in primary vent 60 or primary vent 62, the hydrophobic membrane(s) 64 in secondary vent or vents 66 plus the seal 42 in lead screw aperture 39 will isolate the liquid to the flooded compartment 30 or 50, as the case mary be, and will prevent the liquid from entering the electronics and mechanical housing 40, thereby protecting the motor of pump 41, and the sensitive electronics package 43 in the electronics and mechanical housing 40.

Preferably, the hydrophobic membranes 64 in primary vents 60, and 62, as well as the hydrophobic membrane or membranes 64 in secondary vent or vents 66, are selected so that a water entry pressure (WEP) of each membrane significantly exceeds a fluid pressure at a selected depth, *i.e*., the depth to which they can reasonably expect to be exposed upon immersion in water. For example, in the case where a test pressure of 35.900 Pa (5.2 psi) is requested (*i.e.,* water pressure at a depth of 3,66 meter (12 feet) below the surface), a selected WEP of approximately 68.900 to 103.000 Pa (10 to 15 psi) provides a preferable design margin.

It is likewise preferable that once a suitable WEP is selected, the hydrophobic membrane is selected from among those providing the highest available air flow rate, in order to achieve, along with the desired water resistance, the ability to equalize pressure across the membrane as rapidly as possible.

It will be apparent to those skilled in the art that modifications and variations may be made to the external infusion device casing of the present invention without departing from the scope of the invention. The present invention covers all such modifications and variations provided they fall within the scope of the attached claims and their legal equivalents.

## Claims

1. An infusion device (20), comprising:
a casing (22) comprising an external wall (24) and a plurality of internal housings, including a first housing (30) enclosing a liquid reservoir (32) and a drive mechanism;
an electronics assembly (43) and a pump assembly (41) provided in a second housing (40) for controlling the drive mechanism to dispense the liquid from the reservoir (32) according to a selected pattern;
a battery (52) provided in a third housing (50);
a first primary vent (60) provided between the first housing (30) and the exterior of the casing (22) for venting the infusion device (20) to atmosphere,;
a second primary vent (62) provided between the third housing (50) and the exterior of the casing (22) for venting the infusion device (20) to atmosphere;
at least one secondary vent (66) provided between said first housing (30) and said second housing (40); and
at least one secondary vent (66) provided between said third housing (50) and said second housing (40);
each of said primary and secondary vents (60, 62, 66) including a hydrophobic barrier (64) allowing passage of gas therethrough while preventing passage of liquid therethrough.

2. The infusion device (20) of claim 1, wherein said liquid reservoir (32) contains insulin.

3. The infusion device (20) of claim 1, wherein said liquid reservoir (32) defines a syringe, comprising a generally tubular liquid storage section (33) and a movable plunger (34).

4. The infusion device (20) of claim 1, wherein said drive mechanism comprises a lead screw (38) and a drive nut (138).

5. The infusion device (20) of claim 1 wherein said second housing (40) is vented to atmosphere via at least one of said secondary vents (66) and at least one of said first and third housings (30, 50).

6. The infusion device (20) of claim 1 wherein said first housing (30) comprises means for a user to access said first housing (30).

7. The infusion device (20) of claim 1, wherein said third housing (50) comprises means for a user to access said third housing (50).

8. The infusion device (20) of claim 1, wherein said second housing (40) is inaccessible by a user.

9. The infusion device (20) of claim 1, wherein said hydrophobic barriers (64) comprise membranes, each said membrane having a pre-selected minimum water entry pressure greater than or equal to approximately 68.900 Pa (10 psi).

10. The infusion device (20) of claim 1, wherein said casing (22) is portable.

11. The infusion device (20) of claim 1, wherein said liquid reservoir (32) is refillable.

12. The infusion device (20) of claim 1, wherein said liquid reservoir (32) is replaceable.

13. The infusion device (20) of claim 1, wherein said drive mechanism extends from said first housing (30) to said second housing (40) via an opening comprising a seal (42).

14. The infusion device (20) of claim 1, wherein said first, second, and third housings (30, 40, 50) are sealed from one another against passage of liquid therebetween.

15. A casing (22) for an external infusion pump, comprising:
an outer wall (24);
a first housing (30) configured to enclose a liquid reservoir (32) and a drive mechanism;
an internal second housing (40) configured to enclose pump components (41) and an electronic control assembly (43);
a third housing (50) configured to enclose at least one battery (52);
a first primary vent (60) provided between the first housing (30) and the exterior of the casing (22) for venting the casing (22) to atmosphere;
a second primary vent (62) provided between the third housing (50) and the exterior of the casing (22) for venting the casing (22) to atmosphere;
at least one secondary vent (66) provided between said first housing (30) and said second housing (40); and
at least one secondary vent (66) provided between said third housing (50) and said second housing (40), wherein
each of said primary and secondary vents (60, 62, 66) contain a hydrophobic barrier (64) allowing air passage therethrough while preventing passage of liquid therethrough.

16. The casing (22) of claim 15, wherein said first housing (30) includes a means for a user to access said first housing (30).

17. The casing (22) of claim 15, wherein said third housing (50) includes a means for a user to access said third housing (50).

18. The casing (22) of claim 15, wherein said second housing is inaccessible by a user.

19. The casing (22) of claim 15, wherein said outer wall (24) is configured to be portable.

20. The casing (22) of claim 15, wherein said second housing (40) is vented to atmosphere via said at least one secondary vent (66) and at least one of said first and third housings (30, 50).

21. The casing (22) of claim 15, wherein said vents (60, 62, 66) include a hydrophobic membrane (66) having a pre-selected water entry pressure between about 68.900 Pa (10 psi) and about 103.000 Pa (15 psi).

22. The casing (22) of claim 15, wherein said first housing (30) is defined by an internal wall (26) housing an aperture (39) configured to pass said drive mechanism therethrough, and sealed by a seal (42).

23. The casing (22) of claim 15, wherein said first housing (30), said second housing (40), and said third housing (50), are sealed from one another against passage of liquid therebetween.

## Patentansprüche

1. Infusionsvorrichtung (20), umfassend:
ein Gehäuse (22) mit einer Außenwand (24) und einer Vielzahl von inneren Gehäusen, enthaltend ein erstes Gehäuse (30), das ein Flüssigkeitsreservoir (32) und einen Antriebsmechanismus umschließt;
eine Elektronikanordnung (23) und eine Pumpenanordnung (41), die in einem zweiten Gehäuse (40) zur Ansteuerung des Antriebsmechanismus zum Abgeben der Flüssigkeit aus dem Reservoir (32) gemäß einem ausgewählten Schema vorgesehen sind;
eine Batterie (52), die in einem dritten Gehäuse (50) vorgesehen ist;
einen ersten Primärauslaß (60), der zwischen dem ersten Gehäuse (30) und dem Äußeren des Gehäuses (32) zum Entlüften der Infusionsvorrichtung (20) zur Atmosphäre vorgesehen ist;
einen zweiten Primärauslaß (62), der zwischen dem dritten Gehäuse (50) und dem Äußeren des Gehäuses (22) zum Entlüften der Infusionsvorrichtung (20) zur Atmosphäre vorgesehen ist;
mindesten einen Sekundärauslaß (62), der zwischen dem ersten Gehäuse (30) und dem zweiten Gehäuse (40) vorgesehen ist; und
mindestens einen Sekundärauslaß (66), der zwischen dem dritten Gehäuse (50) und dem zweiten Gehäuse (40) vorgesehen ist;
wobei jeder der Primär- und Sekundärauslässe (60, 62, 66) eine hydrophobe Barriere (64) enthält, die einen Durchgang von Gas durch selbige ermöglicht, aber einen Durchgang von Flüssigkeit durch selbige verhindert.

2. Infusionsvorrichtung (20) nach Anspruch 1, **dadurch gekennzeichnet, dass** das Flüssigkeitsreservoir (32) Insulin enthält.

3. Infusionsvorrichtung (20) nach Anspruch 1, **dadurch gekennzeichnet, dass** das Flüssigkeitsreservoir (32) eine Spritze definiert, die einen im allgemeinen rohrförmigen Flüssigkeitsaufnahmeabschnitt (33) und einen beweglichen Kolben (34) umfasst.

4. Infusionsvorrichtung (20) nach Anspruch 1, **dadurch gekennzeichnet, dass** der Antriebsmechanismus eine Gewindespindel (38) und eine Triebmutter (138) aufweist.

5. Infusionsvorrichtung (20) nach Anspruch 1, **dadurch gekennzeichnet, dass** das zweite Gehäuse (40) zur Atmosphäre über mindestens einen der Sekundärauslässe (66) und mindestens eines der ersten und dritten Gehäuse (30, 50) entlüftet ist.

6. Infusionsvorrichtung (20) nach Anspruch 1, **dadurch gekennzeichnet, dass** das erste Gehäuse (30) ein Mittel für einen Zugriff auf das erste Gehäuse (30) durch einen Benutzer aufweist.

7. Infusionsvorrichtung (20) nach Anspruch 1, **dadurch gekennzeichnet, dass** das dritte Gehäuse (50) ein Mittel für einen Zugriff auf das dritte Gehäuse (50) durch einen Benutzer aufweist.

8. Infusionsvorrichtung (20) nach Anspruch 1, **dadurch gekennzeichnet, dass** das zweite Gehäuse (40) für einen Benutzer unzugänglich ist.

9. Infusionsvorrichtung (20) nach Anspruch 1, **dadurch gekennzeichnet, dass** die hydrophoben Barrieren (64) Membranen aufweisen, wobei jede Membran einen vorab ausgewählten minimalen Wassereintrittsdruck aufweist, der größer als oder gleich näherungsweise 68,900 Pa (10 psi) ist.

10. Infusionsvorrichtung (20) nach Anspruch 1, **dadurch gekennzeichnet, dass** das Gehäuse (22) tragbar ist.

11. Infusionsvorrichtung (20) nach Anspruch 1, **dadurch gekennzeichnet, dass** das Flüssigkeitsreservoir (32) nachfüllbar ist.

12. Infusionsvorrichtung (20) nach Anspruch 1, **dadurch gekennzeichnet, dass** das Flüssigkeitsreservoir (32) austauschbar ist.

13. Infusionsvorrichtung (20) nach Anspruch 1, **dadurch gekennzeichnet, dass** sich der Antriebsmechanismus vom ersten Gehäuse (30) zum zweiten Gehäuse (40) über eine Öffnung mit einer Dichtung (42) erstreckt.

14. Infusionsvorrichtung (20) nach Anspruch 1, **dadurch gekennzeichnet, dass** die ersten, zweiten und dritten Gehäuse (30, 40, 50) gegen Durchgang von Flüssigkeit zwischen selbigen voneinander abgedichtet sind.

15. Gehäuse (22) für eine externe Infusionspumpe, umfassend:
eine Außenwand (24),
ein erstes Gehäuse (30), das so konfiguriert ist, dass es ein Flüssigkeitsreservoir (32) und einen Antriebsmechanismus umschließt;
ein inneres zweites Gehäuse (40), das so konfiguriert ist, dass es Pumpenkomponenten (41) und eine elektronische Steueranordnung (43) umschließt;
ein drittes Gehäuse (50), das so konfiguriert ist, dass es mindestens eine Batterie (52) umschließt;
einen ersten Primärauslaß (60), der zwischen dem ersten Gehäuse (30) und dem Äußeren des Gehäuses (22) zum Entlüften des Gehäuses (22) zur Atmosphäre vorgesehen ist;
einen zweiten Primärauslaß (62), der zwischen dem dritten Gehäuse (50) und dem Äußeren des Gehäuses (22) zum Entlüften des Gehäuses (22) zur Atmosphäre vorgesehen ist;
mindestens einen Sekundärauslaß (66), der zwischen dem ersten Gehäuse (30) und dem zweiten Gehäuse (40) vorgesehen ist; und
mindestens einen Sekundärauslaß (66), der zwischen dem dritten Gehäuse (50) und dem zweiten Gehäuse (40) vorgesehen ist, wobei jeder der Primär- und Sekundärauslässe (60, 62, 66) eine hydrophobe Barriere (64) enthält, die einen Durchgang von Luft durch selbige ermöglicht, aber einen Durchgang von Flüssigkeit durch selbige verhindert.

16. Gehäuse (22) nach Anspruch 15, **dadurch gekennzeichnet, dass** das erste Gehäuse (30) ein Mittel für einen Zugriff auf das erste Gehäuse (30) durch einen Benutzer enthält.

17. Gehäuse (22) nach Anspruch 15, **dadurch gekennzeichnet, dass** das dritte Gehäuse (50) ein Mittel für einen Zugriff auf das dritte Gehäuse (50) durch einen Benutzer enthält.

18. Gehäuse (22) nach Anspruch 15, **dadurch gekennzeichnet, dass** das zweite Gehäuse für einen Benutzer unzugänglich ist.

19. Gehäuse (22) nach Anspruch 15, **dadurch gekennzeichnet, dass** die Außenwand (24) tragbar konfiguriert ist.

20. Gehäuse (22) nach Anspruch 15, **dadurch gekennzeichnet, dass** das zweite Gehäuse (40) zur Atmosphäre über den mindestens einen Sekundärauslaß (66) und mindestens eines der ersten und zweiten Gehäuse (30, 50) entlüftet ist.

21. Gehäuse (22) nach Anspruch 15, **dadurch gekennzeichnet, dass** die Auslässe (60, 62, 66) eine hydrophobe Membran (66) mit einem vorab ausgewählten Wassereintrittsdruck zwischen ungefähr 68,900 Pa (10 psi) und ungefähr 103000 Pa (15 psi) enthalten.

22. Gehäuse (22) nach Anspruch 15, **dadurch gekennzeichnet, dass** das erste Gehäuse (30) durch eine Innenwand (26), die eine Öffnung (39) enthält, die derart konfiguriert ist, dass sie den Antriebsmechanismus hindurch lässt, gebildet und durch eine Dichtung (42) abgedichtet ist.

23. Gehäuse (22) nach Anspruch 15, **dadurch gekennzeichnet, dass** das erste Gehäuse (30), das zweite Gehäuse (40) und das dritte Gehäuse (50) gegen Durchgang von Flüssigkeit zwischen selbigen voneinander abgedichtet sind.

## Revendications

1. Dispositif de perfusion (20), comprenant :
◆ une enveloppe (22) comprenant une paroi externe (24) et une pluralité de boîtiers internes, comprenant un premier boîtier (30) enfermant un réservoir de liquide (32) et un mécanisme d'entraînement ;
◆ un ensemble de commande électronique (43) et un ensemble de pompe (41) prévus dans un deuxième boîtier (40) pour contrôler le mécanisme d'entraînement afin de distribuer le liquide du réservoir (32) selon un modèle sélectionné ;
◆ une batterie (52) prévue dans un troisième boîtier (50) ;
◆ une première aération principale (60) prévue entre le premier boîtier (30) et l'extérieur de l'enveloppe (22) pour mettre le dispositif de perfusion (20) à l'air libre ;
◆ une deuxième aération principale (62) prévue entre le troisième boîtier (50) et l'extérieur de l'enveloppe (22) pour mettre le dispositif de perfusion (20) à l'air libre ;
◆ au moins une aération secondaire (66) prévue entre ledit premier boîtier (30) et ledit deuxième boîtier (40) ; et
◆ au moins une aération secondaire (66) prévue entre ledit troisième boîtier (50) et ledit deuxième boîtier (40) ;
chacune desdites aérations principales et secondaires (60, 62, 66) comprenant une barrière hydrophobe (64) permettant le passage du gaz à travers celle-ci, tout en empêchant le passage du liquide à travers celle-ci.

2. Dispositif de perfusion (20) selon la revendication 1, dans lequel ledit réservoir de liquide (32) contient de l'insuline.

3. Dispositif de perfusion (20) selon la revendication 1, dans lequel ledit réservoir de liquide (32) définit une seringue, comprenant une section de stockage de liquide généralement tubulaire (33) et un piston plongeur mobile (34).

4. Dispositif de perfusion (20) selon la revendication 1, dans lequel ledit mécanisme d'entraînement comprend une vis mère (38) et un écrou d'entraînement (138).

5. Dispositif de perfusion (20) selon la revendication 1, dans lequel ledit deuxième boîtier (40) est mis à l'air libre via au moins l'une desdites aérations secondaires (66) et au moins l'un parmi lesdits premier et troisième boîtiers (30, 50).

6. Dispositif de perfusion (20) selon la revendication 1, dans lequel ledit premier boîtier (30) comprend des moyens pour qu'un utilisateur ait accès audit premier boîtier (30).

7. Dispositif de perfusion (20) selon la revendication 1, dans lequel ledit troisième boîtier (50) comprend des moyens pour qu'un utilisateur ait accès audit troisième boîtier (50).

8. Dispositif de perfusion (20) selon la revendication 1, dans lequel ledit deuxième boîtier (40) est inaccessible par un utilisateur.

9. Dispositif de perfusion (20) selon la revendication 1, dans lequel lesdites barrières hydrophobes (64) comprennent des membranes, chacune desdites membranes ayant une pression d'entrée d'eau présélectionnée supérieure ou égale à approximativement 68,900 Pa (10 psi).

10. Dispositif de perfusion (20) selon la revendication 1, dans lequel ladite enveloppe (22) est portative.

11. Dispositif de perfusion (20) selon la revendication 1, dans lequel ledit réservoir de liquide (32) est rechargeable.

12. Dispositif de perfusion (20) selon la revendication 1, dans lequel ledit réservoir de liquide (32) est remplaçable.

13. Dispositif de perfusion (20) selon la revendication 1, dans lequel ledit mécanisme d'entraînement s'étend dudit premier boîtier (30) audit deuxième boîtier (40) via une ouverture comprenant un joint d'étanchéité (42).

14. Dispositif de perfusion (20) selon la revendication 1, dans lequel lesdits premier, deuxième et troisième boîtiers (30, 40, 50) sont étanches les uns par rapport aux autres, contre le passage du liquide entre eux.

15. Enveloppe (22) pour une pompe de perfusion externe, comprenant :
◆ une paroi externe (24) ;
◆ un premier boîtier (30) configuré pour enfermer un réservoir de liquide (32) et un mécanisme d'entraînement ;
◆ un deuxième boîtier interne (40) configuré pour enfermer des composants de pompe (41) et un ensemble de commande électronique (43) ;
◆ un troisième boîtier (50) configuré pour enfermer au moins une batterie (52) ;
◆ une première aération principale (60) prévue entre le premier boîtier (30) et l'extérieur de l'enveloppe (22) pour mettre l'enveloppe (22) à l'air libre ;
◆ une deuxième aération principale (62) prévue entre le troisième boîtier (50) et l'extérieur de l'enveloppe (22) pour mettre l'enveloppe (22) à l'air libre ;
◆ au moins une aération secondaire (66) prévue entre ledit premier boîtier (30) et ledit deuxième boîtier (40) ; et
◆ au moins une aération secondaire (66) prévue entre ledit troisième boîtier (50) et ledit deuxième boîtier (40), dans lequel :
◆ chacune desdites aérations principales et secondaires (60, 62, 66) contient une barrière hydrophobe (64) permettant le passage de l'air à travers celle-ci, tout en empêchant le passage du liquide à travers celle-ci.

16. Enveloppe (22) selon la revendication 15, dans laquelle ledit premier boîtier (30) comprend des moyens pour qu'un utilisateur ait accès audit premier boîtier (30).

17. Enveloppe (22) selon la revendication 15, dans laquelle ledit troisième boîtier (50) comprend des moyens pour qu'un utilisateur ait accès audit troisième boîtier (50).

18. Enveloppe (22) selon la revendication 15, dans laquelle ledit deuxième boîtier est inaccessible par un utilisateur.

19. Enveloppe (22) selon la revendication 15, dans laquelle ladite paroi externe (24) est configurée de manière à être portative.

20. Enveloppe (22) selon la revendication 15, dans laquelle ledit deuxième boîtier (40) est mis à l'air libre via ladite au moins une aération secondaire (66) et au moins l'un parmi lesdits premier et troisième boîtiers (30, 50).

21. Enveloppe (22) selon la revendication 15, dans laquelle lesdites aérations (60, 62, 66) comprennent une membrane hydrophobe (66) ayant une pression d'entrée d'eau présélectionnée comprise entre environ 68,900 Pa (10 psi) et environ 103 000 Pa (15 psi).

22. Enveloppe (22) selon la revendication 15, dans laquelle ledit premier boîtier (30) est défini par une paroi interne (26) logeant une ouverture (39) configurée pour faire passer ledit mécanisme d'entraînement à travers celle-ci, et hermétiquement fermée par un joint d'étanchéité (42).

23. Enveloppe (22) selon la revendication 15, dans lequel ledit premier boîtier (30), ledit deuxième boîtier (40) et ledit troisième boîtier (50) sont étanches les uns par rapport aux autres, contre le passage du liquide entre eux.
